# EUROPEAN PATENT APPLICATION

(11) **EP 4 257 151 A1**
(43) Date of publication of application: **11.10.2023**
(21) Application number: 21903710.8
(22) Date of filing: 29.11.2021
(51) Int. Cl.: A61K 47/54, A61K 49/22, A61P 35/00, A61K 39/00

(54) **IMMUNE CHECKPOINT INHIBITOR CONJUGATED WITH ULTRASONIC SENSITIZER, AND USE THEREOF**

(30) Priority: 07.12.2020 KR 20200169867
(71) Applicant: IMGT Co, Ltd., Seongnam-si, Gyeonggi-do 13605 (KR)
(72) Inventor: KIM, Daehyun, Seongnam-si, Gyeonggi-do 13604 (KR); KIM, Yoon-Seok, Gwangju-si Gyeonggi-do 12820 (KR); KIM, Hyun Ryoung, Seongnam-si Gyeonggi-do 13581 (KR); JUNG, Eun Ah, Seoul 06277 (KR)
(74) Representative: Dehns
(86) International application number: PCT/KR2021/017744
(87) International publication number: WO 2022/124664

(57) **Abstract**

The present invention relates to an immune checkpoint inhibitor conjugated with an sonosensitizer, a use thereof, and the like. The immune checkpoint inhibitor conjugate conjugated with an sonosensitizer according to the present invention has selectivity for tumors with respect to targeted therapy and specifically responds to ultrasonic waves, and thus can be used to produce drugs for diagnosing and treating cancer. Particularly, the conjugate of the present invention exhibits excellent solubility and stability in an aqueous solution, and provides excellent anticancer effects as compared with simple immune checkpoint inhibitors, and therefore, is expected to be useful as a novel drug for treating cancer.

## Description

### [Technical Field]

The present invention relates to an immune checkpoint inhibitor conjugated with a sonosensitizer and its uses.

This application claims the priority of Korean Patent Application No. 10-2020-0169867, filed on December 7, 2020, and all contents disclosed in the specification and drawings of said application are incorporated herein by reference.

### [Background Art]

Cancer is a major disease accounting for the highest mortality rate in modern society, and representative cancer treatments include surgical intervention, biological therapy, radiation therapy, and chemotherapy using administration of anticancer agents. Recently, immunotherapy, which utilizes the immune system to treat cancer differently from conventional cancer treatments, has gained prominence.

Unlike conventional anticancer drugs that directly attack the cancer itself, immuno-oncology is a method of stimulating the immune system by injecting artificial immune proteins into the body to induce immune cells to selectively attack only cancer cells, and can be broadly divided into passive and active immunotherapy. Passive immunotherapy includes immune checkpoint inhibitors, immune cell therapy, and therapeutic antibodies. Among them, immune checkpoint inhibitors are drugs that activate T cells to attack cancer cells by blocking the activation of immune checkpoint proteins involved in T cell inhibition, such as CTLA-4, PD-1, and PD-L1 inhibitors. In 2016, a PD-L1 antibody drug (atezolizumab) was approved by the FDA for cancer treatment, but immune checkpoint inhibitors have limited therapeutic effects as monotherapy. In addition, active immunotherapy includes cancer vaccines and immune-modulating agents, among which cancer vaccines are drugs that are manufactured from cancer cells or cancer cell-derived substances and injected into the body to trigger the body's natural defense system. However, cancer therapeutic vaccines are complicated to produce and difficult to apply to various types of cancer, and because they are personalized therapies, they impose a financial burden on patients.

Meanwhile, there is a growing interest in materials that can diagnose and treat cancer at the same time. Unlike the traditional method where diagnosis and treatment are performed separately, if treatment is performed at the same time as diagnosis, the location of the disease site can be accurately identified through imaging, and secondary treatment can be performed immediately, resulting in better treatment efficiency. In addition, it is possible to monitor whether the treatment is working properly by imaging the cancer region during treatment, so it is an area of focus in the development of next-generation drugs. Among them, ultrasound imaging is a useful technique for imaging blood vessels or the anatomical structures of tissues and organs, and has the advantages of easy patient accessibility, harmlessness, and low cost.

Against this background, the present inventors have made a conscientious effort to develop a material capable of diagnosing and simultaneously treating cancer by combining an immune checkpoint inhibitor with an sonosensitizer, and have developed a method for combining an immune checkpoint inhibitor with an sonosensitizer, porphyrin, and have confirmed that the conjugate prepared using the above method can be used for diagnosis and treatment of cancer, thereby completing the present invention.

### [Disclosure]

### [Technical Problem]

The inventors of the present invention have made efforts to provide a conjugate and its manufacturing method that overcomes the problems of conventional immune checkpoint inhibitors. The aim is to provide a conjugate with excellent stability and solubility, no precipitation, and excellent cancer cell inhibitory effects, as well as its manufacturing method.

Accordingly, an object of the present invention is to provide a conjugate having tumor selectivity for targeted therapy, wherein the conjugate combines an immune checkpoint inhibitor that inhibits ligands expressed on the surface of cancer cells; and a sonosensitizer.

Another object of the present invention is to provide a sonodynamic composition comprising the conjugate as an active ingredient.

Yet another object of the present invention is to provide a manufacturing method for the conjugate, which comprises the step of combining an immune checkpoint inhibitor with a sonosensitizer using a linker.

However, the technical objects which the present invention intends to achieve are not limited to the technical objects which have been mentioned above, and other technical objects which have not been mentioned will be apparently understood by a person with ordinary skill in the art to which the present invention pertains from the following description.

### [Technical Solution]

To achieve the objects of the present invention, the present invention provides a conjugate having tumor selectivity for targeted therapy, wherein the conjugate is a combination of an immune checkpoint inhibitor that inhibits ligands expressed on the surface of cancer cells; and sonosensitizer.

In one embodiment of the present invention, the immune checkpoint inhibitor may be a PD-L1 inhibitor, but is not limited thereto.

In another embodiment of the present invention, the PD-L1 inhibitor may be one or more selected from the group consisting of atezolizumab, avelumab, durvalumab, envafolimab, cosibelimab, AUNP12, BMS-936559, CS-1001, SHR-1316 (HTI-1088), CBT-502 (TQB-1450), and BGB-A333, but is not limited thereto.

In yet another embodiment of the present invention, the PD-L1 inhibitor may include an amine group, but is not limited thereto.

In yet another embodiment of the present invention, the sonosensitizer may include a double bond, but is not limited thereto.

In yet another embodiment of the present invention, the sonosensitizer may be one or more selected from the group consisting of porphyrins, porphyrin isomers, and expanded porphyrins, but is not limited thereto.

In yet another embodiment of the present invention, the sonosensitizer may include one or more selected from the group consisting of amine groups (-NH₂), hydroxyl groups (-OH), carboxyl groups (-COOH), and sulfate groups (-SO₄), but is not limited thereto.

In yet another embodiment of the present invention, the conjugate may include the structure of Chemical Formula 1, but is not limited thereto.

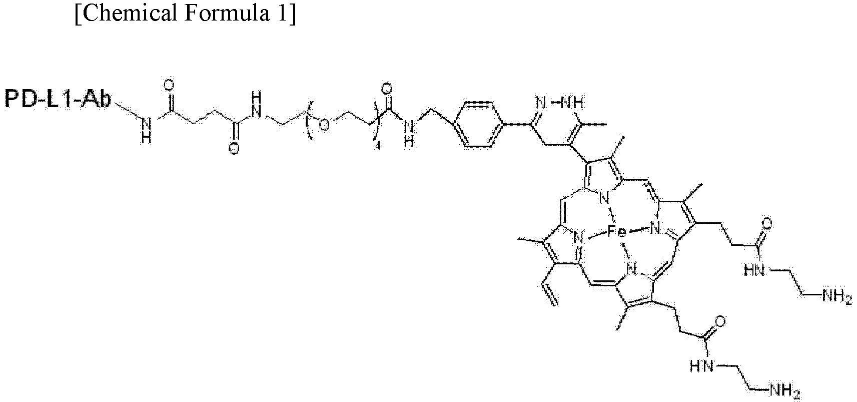

In yet another embodiment of the present invention, the conjugate may have the immune checkpoint inhibitor and the sonosensitizer combined at a ratio of 1:1 to 1:10, but is not limited thereto.

Furthermore, the present invention provides a sonodynamic composition comprising the conjugate as an active ingredient.

In one embodiment of the present invention, the composition may be for cancer diagnosis and treatment, but is not limited thereto.

Furthermore, the present invention provides a method for producing the conjugate, which includes the step of combining the immune checkpoint inhibitor with the sonosensitizer using a linker.

In the present invention, the combining step may include, but is not limited to:
i) a step of reacting the immune checkpoint inhibitor with a linker to produce a first conjugate; and
ii) a step of reacting the first conjugate with a sonosensitizer to produce the final conjugate.

In the present invention, the linker may include one or more selected from the group consisting of PEG (polyethylene glycol), poloxamer, polyvinylpyrrolidone, and polyoxazoline, but is not limited thereto.

In the present invention, the linker may be methylenetetrazine-PEG4-NHS, but is not limited thereto.

The present invention also provides methods for diagnosis and treatment of cancer, comprising the step of administering to a subject a composition comprising as an active ingredient a tumor-selective conjugate or a pharmaceutically acceptable salt thereof for targeted therapy, which combines an immune checkpoint inhibitor that inhibits a ligand expressed on the surface of a cancer cell; and a sonosensitizer.

Moreover, the present invention provides diagnostic and therapeutic uses of a composition comprising as an active ingredient a tumor-selective conjugate or pharmaceutically acceptable salt thereof for targeted therapy in combination with an immune checkpoint inhibitor that inhibits a ligand expressed on the surface of a cancer cell; and a sonosensitizer.

In addition, the present invention provides uses for preparing compositions for diagnosis and treatment of cancer comprising as an active ingredient a tumor-selective conjugate or pharmaceutically acceptable salt thereof for targeted therapy in combination with an immune checkpoint inhibitor that inhibits a ligand expressed on the surface of a cancer cell; and a sonosensitizer.

### [Advantageous Effects]

The conjugate of the present invention has tumor selectivity for targeted therapy and specifically responds to ultrasound, making it suitable for use in the manufacture of pharmaceuticals for cancer treatment. In particular, the conjugate of the present invention demonstrates excellent solubility and stability in aqueous solutions while providing superior anticancer effects compared to simple immune checkpoint inhibitors, and is expected to be usefully employed as a novel drug for cancer treatment.

### [Description of the Drawings]

FIG. 1 illustrates the structure of porphyrin-EDA and the purity measured by HPLC-UV.
FIG. 2 illustrates a chromatogram representing the molecular weight of PD-L1 antibody, primary conjugate, and ICI-SDT as measured by MALDI-TOF.
FIG. 3 illustrates the chemical structure and images after filtering for each material corresponding to Synthesis Nos 1, 2, and 3 in Table 2.
FIG. 4 illustrates a confocal image representing the binding affinity of ICI-SDT.
FIG. 5 illustrates data on the ROS generation capability of ICI-SDT.
FIG. 6 illustrates cell survival rate experimental data depending on the presence or absence of porphyrin-EDA and ultrasound.

### [Best Modes of the Invention]

The present invention provides a conjugate having tumor selectivity for targeted therapy, wherein the conjugate combines an immune checkpoint inhibitor that inhibits ligands expressed on the surface of cancer cells; and an ultrasound sensitizing agent.

Hereinafter, the present invention will be described in detail.

In the present invention, the immune checkpoint inhibitor may be a PD-L1 inhibitor, but is not limited thereto.

In the present invention, the PD-L1 inhibitor may be an anti-PD-L1 antibody, but is not limited thereto.

In the present invention, the PD-L1 inhibitor may be one or more selected from the group consisting of atezolizumab, avelumab, durvalumab, envafolimab, cosibelimab, AUNP12, BMS-936559, CS-1001, SHR-1316 (HTI-1088), CBT-502 (TQB-1450), and BGB-A333, but is not limited thereto.

In the present invention, the sonosensitizer may be one or more selected from the group consisting of porphyrins, porphyrin isomers, and expanded porphyrins, but is not limited thereto.

In the present invention, the porphyrins, porphyrin isomers, and expanded porphyrins may be one or more selected from the group consisting of chlorin e6, photodithazine, Radachlorin, 2-(1-hexylethyl)-2-devinylpyropheophorbide-α (HPPH), zinc phthalocyanine (ZnPc), pheophorbide a compounds, and phorphyrin compounds, but is not limited thereto.

In the present invention, an ethylenediamine (EDA) having an amine group was reacted with the terminal of the porphyrin, which is a sonosensitizer, to synthesize a water-soluble porphyrin (refer to Example 2 of the present invention).

In the present invention, the terminal of sonosensitizer may include one or more selected from the group consisting of amine groups (-NH₂), hydroxyl groups (-OH), carboxyl groups (-COOH), and sulfate groups (-SO₄), but is not limited thereto.

In the present invention, the conjugate may comprise a linker or a fragment of the linker, but is not limited thereto.

In the present invention, the linker may include one or more selected from the group consisting of PEG (polyethylene glycol), poloxamer, polyvinylpyrolidone, and polyoxazoline, but is not limited thereto.

In the present invention, PEG refers to polyethylene glycol residues, and the average molecular weight may be 200 to 100,000, 500 to 50,000, about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 25,000, 30,000, 35,000, 40,000, 45,000, 50,000, 55,000, 60,000, 65,000, 70,000, 75,000, 80,000, 85,000, 90,000, 95,000, or 100,000 kDa. In the present invention, PEG may be PEG4, but is not limited thereto.

In the present invention, the poloxamer is a nonionic triblock copolymer with hydrophilic ethylene oxide groups at both ends and a hydrophobic propylene oxide group in the center. The poloxamer exhibits temperature sensitivity, enabling sol-gel transition depending on the concentration and temperature, and its properties vary depending on the ratio of polyoxypropylene and polyoxyethylene. The poloxamer may be selected from a group consisting of poloxamer 101, poloxamer 105, poloxamer 105 benzoate, poloxamer 108, poloxamer 122, poloxamer 123, poloxamer 124, poloxamer 181, poloxamer 182, poloxamer 182 dibenzoate, poloxamer 183, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 331, poloxamer 333, poloxamer 334, poloxamer 335, poloxamer 338, poloxamer 401, poloxamer 402, poloxamer 403, and poloxamer 407, but is not limited thereto.

In the present invention, polyoxazoline (POZ) is a polymer prepared from 2-substituted-2-oxazoline monomers. The polymer is water-soluble and has been reported to be non-toxic in mammalian model systems.

In the present invention, the linker may be methylenetetrazine-PEG4-NHS, but is not limited thereto. The methylenetetrazine of the linker reacts with the double bond of porphyrin-EDA, and the NHS group reacts with the amine group of the antibody, thereby forming the conjugate (refer to the examples of the present invention).

In the present invention, the conjugate may include the structure of Chemical Formula 1 below, but is not limited thereto.

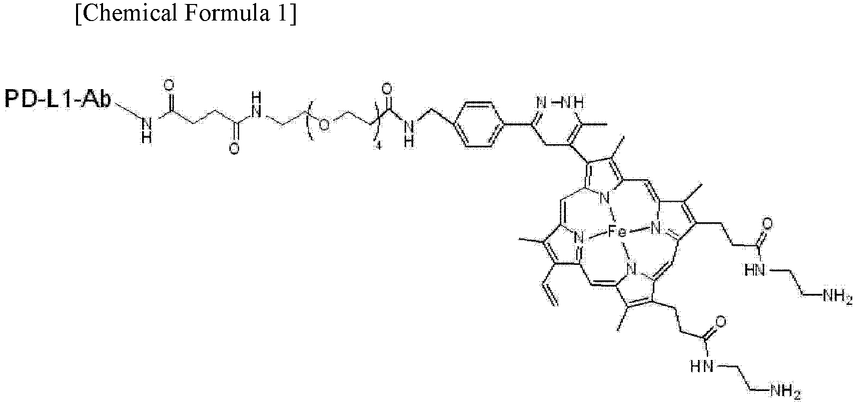

In the present invention, the conjugate may be combined at a ratio of 1:1 to 10, 1:1 to 9, 1:1 to 8, 1:1 to 7, 1:1 to 6, 1:2 to 10, 1:2 to 9, 1:2 to 8, 1:2 to 7, 1:2 to 6, 1:3 to 10, 1:3 to 9, 1:3 to 8, 1:3 to 7, 1:3 to 6, 1:4 to 10, 1:4 to 9, 1:4 to 8, 1:4 to 7, 1:4 to 6, 1:4.5 to 6, or 1:5 to 6 of an immune checkpoint inhibitor and a sonosensitizer, but is not limited thereto.

In addition, the present invention provides a sonodynamic composition comprising the conjugate as an active ingredient.

In this specification, sonodynamics refers to a phenomenon where the cytotoxic activity exhibited by a sonosensitizer is enhanced by ultrasound. That is, it involves focusing ultrasound energy on a deeply embedded malignant site and locally activating the sonosensitizer injected in advance. The mechanism of sonodynamic therapy includes the generation of radicals induced by the sonosensitizer causing sequential peroxidation of membrane lipids by peroxides or alkoxy radicals, conferring physical instability to the cell membrane by the sonosensitizer making the cell more sensitive to shear forces, or increased drug mobility through the cell membrane by ultrasound (sonoporation).

In the present invention, the term "ultrasound" is generally used to refer to sound waves with frequencies exceeding the audible frequency range of 16 Hz to 20 kHz, and high-intensity focused ultrasound introduces focused ultrasound that provides continuous, high-intensity ultrasound energy to the focal point, which can generate instantaneous thermal effects (65-100°C), cavitation effects, mechanical effects, and sonochemical effects depending on the energy and frequency. Ultrasound is harmless when passing through human tissue, but high-intensity focused ultrasound generates sufficient energy to cause coagulative necrosis and thermal ablation effects, regardless of the type of tissue. In the present invention, the ultrasound refers to sound waves with frequencies greater than the audible frequency range of 16 Hz to 20 kHz.

Photodynamic Therapy (PDT) has received considerable attention due to its invasiveness and site-specific activation. PDT directly induces cancer cell death by using light to activate a photosensitizer that generates reactive oxygen species (ROS). However, near-infrared (NIR) lasers have limitations in tissue penetration, and they stimulate all photosensitizers present in the propagation path. To overcome these limitations, the development of sonodynamic therapy technology using ultrasound (US) instead of light has been demanded. In the case of sonodynamic therapy, the tissue attenuation coefficient is low and non-radiative; therefore, ultrasound penetrates much deeper into body tissues. Additionally, it harmlessly passes through multiple layers of tissue, and only stimulates the sensitizer at the focal point with high spatial precision.

In the present invention, the composition may be for diagnosis and treatment of cancer, but is not limited thereto.

Generally, a tumor refers to an abnormal mass resulting from the autonomous excessive growth of body tissue, and tumors can be classified into benign tumors and malignant tumors. Malignant tumors grow much faster than benign tumors, invading surrounding tissues and causing metastasis, thereby posing a threat to life. Such malignant tumors are commonly referred to as 'cancer.'

In the present invention, the type of cancer is not particularly limited. Nonlimiting examples of the cancer include cervical cancer, lung cancer, pancreatic cancer, non-small cell lung cancer, liver cancer, colon cancer, bone cancer, skin cancer, head cancer, neck cancer, melanoma, intraocular melanoma, uterine cancer, ovarian cancer, rectal cancer, brain tumor, bladder cancer, blood cancer, stomach cancer, perianal cancer, breast cancer, fallopian tube cancer, endometrial cancer, vaginal cancer, vulvar cancer, Hodgkin's disease, esophageal cancer, small intestine cancer, endocrine gland cancer, thyroid cancer, parathyroid cancer, adrenal cancer, connective tissue sarcoma, urethral cancer, penile cancer, prostate cancer, kidney cancer, ureter cancer, renal cell carcinoma, renal pelvis carcinoma, central nervous system (CNS) tumors, primary CNS lymphoma, spinal cord tumors, brainstem glioma, or pituitary adenoma. Preferably, the cancer may be breast cancer, but it is not limited thereto.

The present invention may also comprise a pharmaceutically acceptable salt of the conjugate as an active ingredient. As used herein, the term "pharmaceutically acceptable salt" includes a salt derived from a pharmaceutically acceptable inorganic acid, organic acid, or base. As used herein, the term "food-acceptable salt" includes a salt derived from a food-acceptable organic acid, inorganic acid, or base. As used herein, the term "veterinally acceptable salt" includes a salt derived from a veterinally acceptable inorganic acid, organic acid, or base.

Suitable acid examples include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, benzenesulfonic acid, and the like. Acid addition salts can be prepared by conventional methods, for example, by dissolving the compound in an excess of an acid aqueous solution and precipitating the salt using a water-miscible organic solvent such as methanol, ethanol, acetone, or acetonitrile. In addition, acid addition salts can be prepared by heating an equimolar amount of the compound and an acid or alcohol in water and subsequently evaporating the mixture to a dried state, or by suction filtration of the precipitated salt.

Salts derived from suitable bases can include alkali metals such as sodium and potassium, alkaline earth metals such as magnesium, and ammonium, but are not limited thereto. Alkali metal or alkaline earth metal salts can be obtained, for example, by dissolving the compound in an excess of alkali metal hydroxide or alkaline earth metal hydroxide solution, filtering the insoluble compound salt, and evaporating and drying the filtrate. In this case, it is pharmaceutically appropriate to prepare sodium, potassium, or calcium salts as metal salts, and corresponding silver salts can be obtained by reacting alkali metal or alkaline earth metal salts with a suitable silver salt (e.g., silver nitrate).

The content of the conjugate in the composition of the present invention can be appropriately adjusted depending on the symptoms of the disease, the progression of the symptoms, the condition of the patient. For example, the content may be 0.0001 to 99.9 weight% or 0.001 to 50 weight% based on the total composition weight, but is not limited thereto. The aforementioned content ratio is based on the dried weight after removing the solvent.

The pharmaceutical composition of the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention is determined depending on the type of a drug, which is an active ingredient, along with various related factors such as a disease to be treated, administration route, the age, gender, and body weight of a patient, and the severity of diseases.

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "improvement" as used herein means all actions that reduce the degree of parameters related to a target disease, e.g., symptoms via administration of the composition according to the present invention.

As used herein, the term "diagnosis" refers to the identification of the presence, extent (symptomatology), and/or characteristics of a pathological condition. As used herein, "diagnosis of cancer" is the identification of a pathological condition of cancer, such as whether or not cancer has developed, the extent of the cancerous area, the location of the cancer (cancer cells), and the extent of the cancer, and it is important to accurately and rapidly distinguish cancer cells or cancer cells and/or tissue from normal cells or normal tissue for a more accurate diagnosis.

In addition to the conjugate, the composition for diagnosing cancer according to the present invention may further comprise elements conventionally used for detecting proteins with antibodies, such as secondary antibodies, substrates for chromogenic reactions of the secondary antibodies, cofactors, and the like.

Additionally, the present invention provides a method for manufacturing the conjugate, which includes a step of coupling an immune checkpoint inhibitor with a sonosensitizer using a linker.

The aspects related to the conjugate can be applied to this manufacturing method.

In the present invention, the manufacturing method may include, but is not limited to, the following steps:
i) reacting the immune checkpoint inhibitor with the linker to produce a primary conjugate; and
ii) reacting the primary conjugate with a sonosensitizer to produce the final conjugate.

In the step i), the immune checkpoint inhibitor and the linker may react in a mass ratio of 1: 0.1 to 20, 1: 1 to 20, 1: 1 to 15, 1: 1 to 10, 1: 3 to 10, 1: 3 to 8, 1: 3 to 6, 1: 4 to 6, or approximately 1: 5, but is not limited thereto.

In the step i), the reaction can be performed in an aqueous solution, but is not limited thereto.

In the step ii), the primary conjugate and the sonosensitizer may react at a molar ratio of 1 to 100:1, 1 to 90:1, 1 to 80:1, 1 to 70:1, 1 to 60:1, 1 to 50:1, 1 to 40:1, 1 to 30:1, 1 to 25:1, 5 to 50:1, 5 to 40:1, 5 to 30:1, 10 to 30:1, 15 to 25:1, or 20:1, but is not limited thereto.

In the step ii), the reaction may be performed under conditions of pH 5 to 10, 6 to 10, 7 to 10, 8 to 10, or 8 to 9, but is not limited thereto.

In the present invention, the linker may include one or more selected from a group consisting of PEG (polyethylene glycol), poloxamer, polyvinylpyrolidone, and polyoxazoline, but is not limited thereto.

In the present invention, the linker may include N-hydroxysuccinimide (NHS) at one terminal end, but is not limited thereto.

In the present invention, the linker may include methylenetetrazine at the other terminal end, but is not limited thereto.

In another embodiment of the present invention, the linker may be methylenetetrazine-PEG4-NHS, but is not limited thereto. The methylenetetrazine of the linker reacts with the double bond of porphyrin-EDA, and the NHS moiety reacts with the amine group of the antibody, thereby forming the conjugate (refer to the examples of the present invention).

Furthermore, the steps are not necessarily limited to the order described herein.

Throughout the entire specification of the present invention, when a part is stated to "include" certain components, it means that it may further include other components, unless specifically stated otherwise. The terms "about," "substantially," and the like used throughout the entire specification of the present invention are used in a meaning close to or at the mentioned values when unique manufacturing and material tolerances are presented and are used to prevent unconscientious infringers from unfairly exploiting precise or absolute numerical disclosures for facilitating understanding of the invention.

Throughout the entire specification of the present invention, the term "combination thereof' included in the Markush-type expression refers to one or more mixtures or combinations selected from a group consisting of the components listed in the Markush-type expression, meaning that it includes one or more selected from the group consisting of the components.

Throughout the specification (especially in the patent claims), the use of the term "the" and similar indicative terms may apply to both singular and plural forms. Also, when a range is mentioned, it includes individual values within the range (unless otherwise stated), as if each individual value constituting the range is mentioned in detail. Finally, unless the steps constituting a method are explicitly stated in order or contrary statements are made, the steps may be performed in an appropriate order. The steps are not necessarily limited to the order described. The use of all examples or exemplary terms (e.g., etc.) is merely for the purpose of describing technical ideas in detail and does not limit the scope of the claims, unless otherwise limited by the claims. Furthermore, those skilled in the art can recognize that various modifications, combinations, and alterations may be designed within the category of the patent claims or equivalents thereof, depending on the design conditions and factors.

The terms such as "first," "second," etc., may be used to describe various components, but the components should not be limited by these terms. These terms are used solely for the purpose of distinguishing one component from another. For example, without departing from the scope of the present invention, the first component may be named the second component, and similarly, the second component may be named the first component. The term "and/or" includes any combination of multiple related listed items or any of the multiple related listed items.

The present invention is subject to various modifications and may have various embodiments, and specific embodiments are exemplified in the drawings and described in detail in the detailed description. However, this is not intended to limit the present invention to any particular embodiment, and should be understood to include all modifications, equivalents, or substitutes within the scope of the ideas and technical scope of the present invention. In describing the present invention, a detailed description of related disclosed technologies may be omitted if it is deemed that the specific description may obscure the gist of the present invention.

Hereinafter, to help in understanding the present invention, exemplary examples will be suggested. However, the following examples are merely provided to more easily understand the present invention, and not to limit the present invention.

### [Examples]

### Example 1. Preparation of Mouse PD-L1 Antibody-PEG4-Methylene Tetrazine Conjugate (Primary Conjugate)

To prepare a mouse PD-L1 antibody combined with methylenetetrazine-PEG4, a reaction between a mouse PD-L1 antibody (BE0101, BioXcell) and methylenetetrazine-PEG4-NHS (Futurechem) was carried out in a 10% sucrose solution at pH 8.8. In 1 mL of the solution, 5 mg of mouse PD-L1 antibody and 25 mg of methylenetetrazine-PEG4-NHS were added to proceed with the reaction. After reacting for 1 hour, the material was placed in an ultra-centrifugal filter (Amicon Ultra-15 30K), and the primary conjugate was concentrated for 15 minutes at 3,200 g, removing residual methylenetetrazine-PEG4-NHS. The concentrated primary conjugate was added to a 10% sucrose solution at pH 8.8 to obtain a concentration of 5 mg/mL and stored. The amount of methylenetetrazine-PEG4 bound to the PD-L1 antibody was measured by analyzing the molecular weight through MALDI-TOF. The results are shown in Table 1.

**[Table 1]**

| The number of methylenetetrazine-PEG4-NHS bound to the PD-L1 antibody calculated by molecular weight | | | |
|---|---|---|---|
| Molecular weight of mouse PD-L1 | Molecular weight of primary conjugate | Increased molecular weight | Number of conjugated methylenetetrazine |
| 148,082 Da | 150,873 Da | 2,791 Da | 5.27 |

### Example 2. Preparation of SDT (Sonodynamic Therapy) Agent (Porphyrin-EDA)

To perform a reaction between mouse PD-L1-PEG4-methylenetetrazine and porphyrin (Hemin, Sigma Aldrich) in an aqueous solution, the synthesis of water-soluble porphyrin (Porphyrin-EDA) was carried out. CDI (1,1'-Carbonyldiimidazole, Sigma Aldrich) was used to synthesize porphyrin-EDA, and DMF was used as the solvent. Porphyrin, EDA (Ethylene diamine, Sigma Aldrich), and CDI were reacted at a molar ratio of 1 :20:2.1 and a concentration of 20 mg/mL. After reacting for 30 minutes, EDA was added and the reaction was carried out for an additional 24 hours. The reaction was terminated by diluting the solvent with distilled water at a 1/8 ratio. The precipitate was obtained by centrifuging at 18,000 rpm for 15 minutes using a centrifuge. The precipitate was dissolved in 50 mM HCl, and the floating matters were removed through a 0.2 µm syringe filter. The porphyrin-EDA contained in the HCl solution was purified using preparative liquid chromatography (LC), and the synthesis was confirmed by verifying the molecular weight of the material through LC/MS. Porphyrin-EDA was freeze-dried under liquid nitrogen at 15Pa and below -20°C, and the yield of the resulting powder was determined to be approximately 91.5% by measuring its mass.

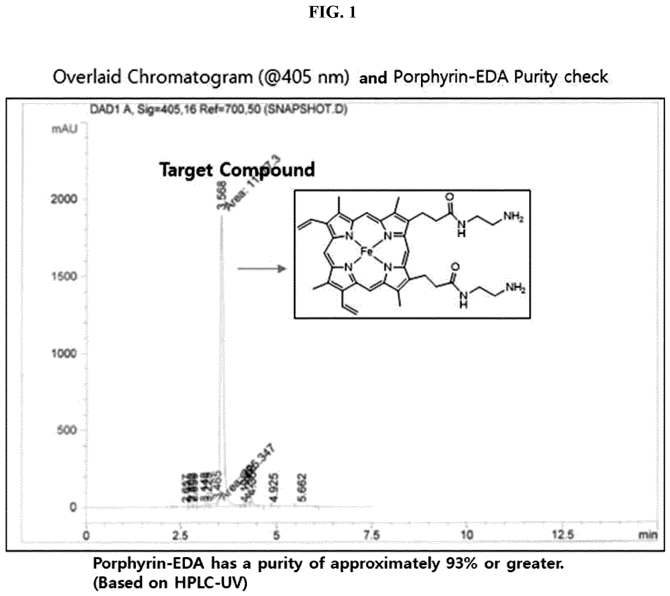

As shown in FIG. 1, the structure of porphyrin-EDA was confirmed, and its purity was determined to be greater than 93%.

### Example 3. Preparation of the Final Conjugate of Immune Checkpoint Inhibitor (ICI)-SDT (Sonodynamic Therapy) Agent (Mouse PD-L1-Antibody-PEG4-Methylene Tetrazine-Porphyrin-EDA)

The primary conjugate and porphyrin-EDA were synthesized, and the synthesized final material was verified by measuring its molecular weight using MALDI-TOF. To do this, the primary conjugate was dissolved in a 10% sucrose solution at pH 8.8, and porphyrin-EDA was added at a 20-fold molar ratio to react for 24 hours. After the reaction, unreacted materials were removed using an ultra-centrifugal filter, and the ICI-SDT agent was concentrated. Subsequently, the floating matters mixed with the ICI-SDT agent were removed using a 0.2 µm syringe filter. The molecular weight of the prepared ICI-SDT was measured using MALDI-TOF, and the amount of bound porphyrin-EDA was quantified through the increased molecular weight.

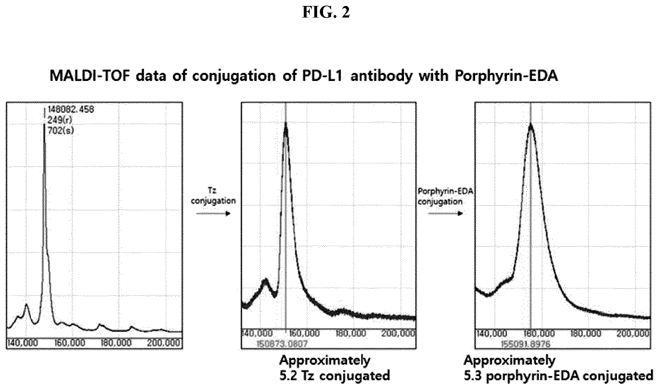

As a result, as shown in FIG. 2, it was confirmed that approximately 5.3 porphyrin-EDA molecules were conjugated to the primary conjugate.

### Example 4. Comparison of the Properties of the Final Product Depending on the Synthesis Method

To compare the properties of the synthesized materials with different methods, a total of three types of binding were selected and experiments were conducted. Synthesis No. 1 involved attaching methylenetetrazine (Tz) to the antibody and TCO (Trans-cyclooctene) to porphyrin, followed by conjugating Tz-TCO using click chemistry. Synthesis No. 2 involved attaching Tz to the antibody and synthesizing it through the double bond conjugation between Tz and porphyrin-EDA. Synthesis No. 3 involved attaching azide to the antibody and alkene to porphyrin, followed by conjugating the antibody and porphyrin using azide-alkene conjugation.

After synthesis, the stability and the number of conjugated porphyrins were compared. The number of conjugated porphyrins is shown in Table 2. In the case of Synthesis No. 2 in Table 2, it is the final conjugate synthesized following Examples 1 to 3.

**[Table 2]**

| Type-specific Binding Experiments and Physicochemical Property Data Between Porphyrin and PD-L1 Antibodies | | | |
|---|---|---|---|
| Synthesis No. | Types of Conjugation | Size | # of porphyrin-EDA (Tz) |
| 1 | PD-L1 Ab-TZ-TCO-Porphyrin | 151,643 | 1.7 |
| 2 | PD-L1 Ab-Tz-Porphyrin-EDA | 154,299 | 5.3 |
| 3 | PD-L1 Ab-AA-Porphyrin | 154,701 | 2.1 |

| | | | |
|---|---|---|---|
| *EDA: Ethylenediamine, AA: Azide & Alkyne conjugation, Tz: Methylenetetrazine, TCO: Trans-cyclooctene, TZ-TCO: conjugation using Tz and TCO | | | |

The final products of Synthesis Nos. 1 and 3 were measured for the number of porphyrin bonds using MALDI-TOF data.

As shown in Table 2, the final products of Synthesis Nos. 1 and 3 had relatively low binding numbers of 1.7 and 2.1, respectively, confirming that the final conjugate prepared according to the present invention exhibited excellent binding ability with porphyrin.

Moreover, after synthesizing Synthesis Nos. 1, 2, and 3, a 0.2 µm filter was used to remove the precipitate of the final product.

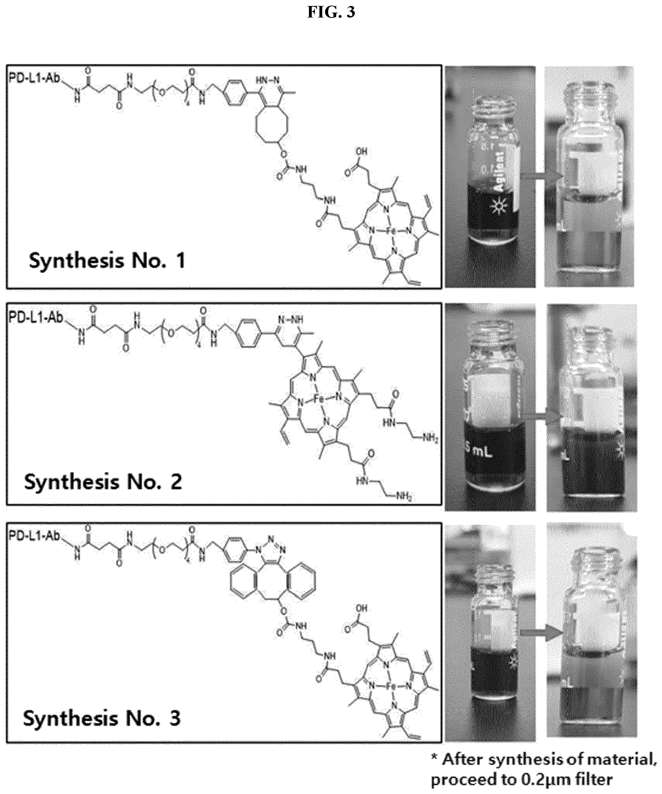

As a result, as shown in FIG. 3, Synthesis Nos. 1 and 3 showed precipitate formation, and after filtering, the color of the material became lighter due to the dilution effect caused by the removal of the precipitate. In contrast, in the case of Synthesis No. 2, the synthesized material was stably formed and had excellent solubility, and no precipitate was formed, maintaining the same appearance even after 0.2 µm filtering.

### Example 5. Confirmation of Binding Affinity of ICI-SDT Agent Conjugate in vitro

To evaluate whether the binding affinity of the PD-L1 antibody remains effective after conjugation with the SDT agent, an in vitro experiment using the 4T1 cell line was conducted. Interferon-γ (IFN-γ) was treated on 4T1 cells to increase the expression level of PD-L1, and the cells were then transferred to a slide for culture. Subsequently, the final synthesized ICI-SDT agent was added, and the cells were cultured for 1 hour. Afterward, the binding affinity to the cells was confirmed through images captured using a confocal scanning microscope.

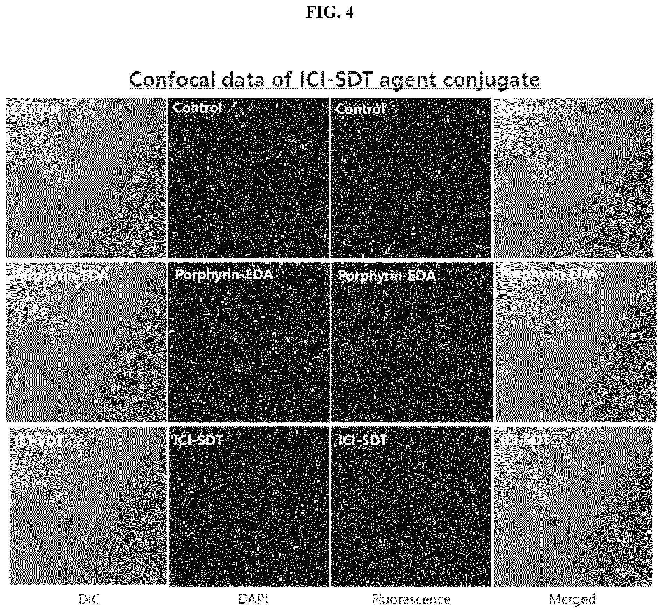

As a result, as shown in FIG. 4, it was confirmed that the binding affinity of the prepared ICI-SDT agent remained effective even after conjugation with the SDT agent, verifying the possibility of targeted therapy for cancer cells.

### Example 6. ROS Generation Test of ICI-SDT Agent and Porphyrin-EDA by Ultrasound

To measure ROS generation by ultrasound, ICI-SDT agent and porphyrin-EDA were exposed to ultrasound and ROS production was measured using DCFH-DA (2',7'-dichlorofluorescin diacetate, Sigma-Aldrich). 20µg of porphyrin-EDA was dissolved in 5mL of distilled water, and 25µM DCFH-DA and 25µM Esterase were added for the experiment. For ultrasound exposure, the Sonidel-100 equipment was set to 3W, 100Hz, and 50%, and ultrasound was applied for 3 minutes. After ultrasound exposure, fluorescence was measured using an ELISA instrument under ex485/em535 conditions.

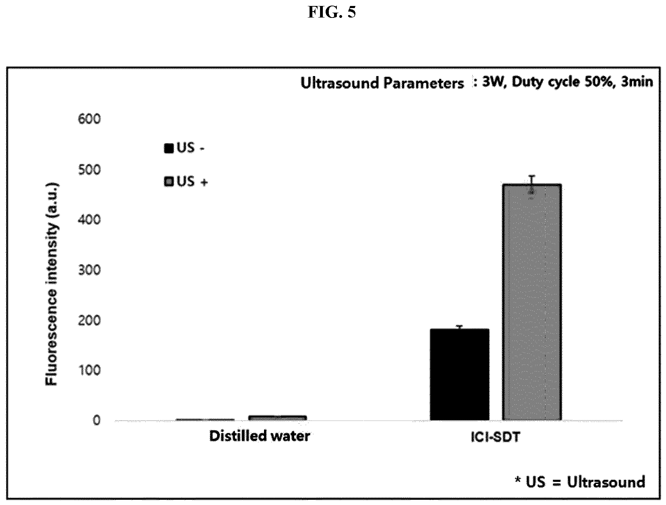

As a result, as shown in FIG. 5, it was confirmed that 2.8 times more ROS was generated for ICI-SDT after ultrasound exposure than before, demonstrating an excellent ROS generation effect for targeted sonodynamic therapy in cancer treatment.

### Example 7. Cell Viability Test for ROS Generated by ICI-SDT

To investigate the effect of ROS generated by porphyrin-EDA upon ultrasound exposure on cell viability, an in vitro cell experiment was conducted. The experimental groups were divided into ultrasound treatment only (US only), antibody and ultrasound treatment (PD-L1+US), porphyrin and ultrasound treatment (PPR+US), antibody, porphyrin and ultrasound treatment (PD-L1+PPR+US), and the conjugate of the invention and ultrasound treatment (ICI-SDT+US), with porphyrin-EDA used at a concentration of 20µg/mL. The ultrasound parameters were set to 1.5W/cm², a duty cycle of 50%, and 1 minute using the Soniel-100 equipment. 1×10⁵ 4T1 breast cancer cells were cultured in a 6-well plate, and after adding the substances to each group, the medium was replaced an hour later to wash off the substances that were not uptaken by the cells. Subsequently, ultrasound was applied, and the cells were cultured overnight before measuring cell viability through an MTT assay.

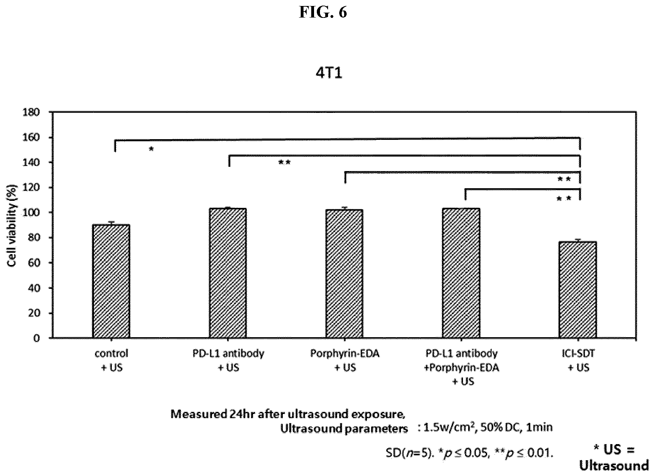

As shown in FIG. 6, since the PD-L1 antibody itself is non-toxic, there was no difference in cell viability between the control, PD-L1 antibody, and PD-L1 antibody+US groups. In contrast, in the ICI-SDT+US group, the chemically conjugated porphyrin-EDA with the PD-L1 antibody formed ROS under ultrasound exposure conditions, significantly affecting cell viability.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

The conjugate of the present invention has selectivity for tumors related to targeted therapy and specifically responds to ultrasound, making it suitable for use in the manufacture of pharmaceuticals for cancer treatment. In particular, the conjugate of the present invention demonstrates excellent solubility and stability in aqueous solutions while providing superior anti-cancer effects compared to simple immune checkpoint inhibitors. Therefore, it can be usefully employed as a new pharmaceutical for cancer treatment, indicating its industrial applicability.

## Claims

1. A conjugate having tumor selectivity for targeted therapy, wherein the conjugate is combination of an immune checkpoint inhibitor that inhibits ligands expressed on the surface of cancer cells; and sonosensitizer.

2. The conjugate of claim 1, wherein the immune checkpoint inhibitor is a PD-L1 inhibitor.

3. The conjugate of claim 2, wherein the PD-L1 inhibitor is one or more selected from the group consisting of atezolizumab, avelumab, durvalumab, envafolimab, cosibelimab, AUNP12, BMS-936559, CS-1001, SHR-1316 (HTI-1088), CBT-502 (TQB-1450), and BGB-A333.

4. The conjugate of claim 1, wherein the sonosensitizer is one or more selected from the group consisting of porphyrin, porphyrin isomers, and expanded porphyrins.

5. The conjugate of claim 1, wherein the sonosensitizer comprises one or more selected from a group consisting of an amino group (-NH₂), a hydroxyl group (-OH), a carboxyl group (-COOH), and a sulfate group (-SO₄).

6. The conjugate of claim 1, wherein the conjugate comprises a structure of Chemical Formula 1.

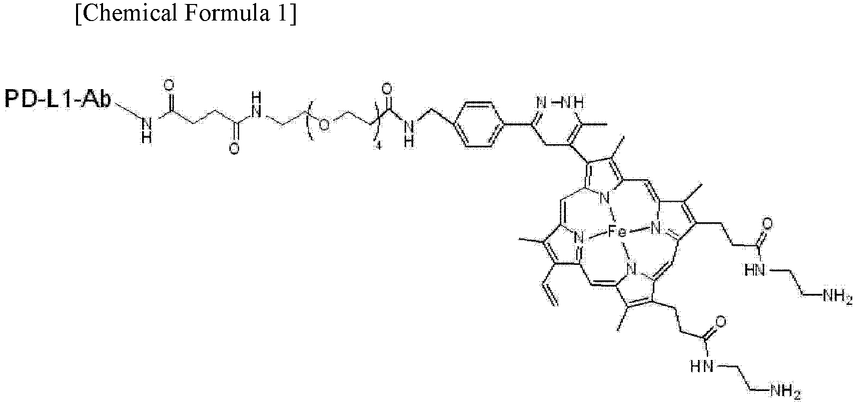

7. The conjugate of claim 1, wherein the conjugate comprises an immune checkpoint inhibitor and the sonosensitizer bound at a ratio of 1:1 to 1:10.

8. A sonodynamic composition, comprising the conjugate of claim 1 as an active ingredient.

9. The composition of claim 8, wherein the composition is for diagnosis and treatment of cancer.

10. A method of manufacturing the conjugate of claim 1, comprising the step of conjugating an immune checkpoint inhibitor to the sonosensitizer using a linker.

11. A method of claim 10, wherein the conjugating step comprises the following steps:
i) reacting the immune checkpoint inhibitor with a linker to produce a primary conjugate; and
ii) reacting the primary conjugate with the sonosensitizer to produce the final conjugate.

12. A method of diagnosing and treating cancer, comprising the step of administering to a subject a composition comprising the conjugate of claim 1 or a pharmaceutically acceptable salt thereof as an active ingredient.

13. A use of the composition comprising the conjugate of claim 1 or a pharmaceutically acceptable salt thereof as an active ingredient for diagnosis and treatment of cancer.

14. A use of the composition comprising the conjugate of claim 1 or a pharmaceutically acceptable salt thereof as an active ingredient for manufacturing a diagnostic and therapeutic agent for cancer.
